# EUROPEAN PATENT APPLICATION

(11) **EP 4 179 951 A1**
(43) Date of publication of application: **17.05.2023**
(21) Application number: 21207467.8
(22) Date of filing: 10.11.2021
(51) Int. Cl.: A61B 1/00, H01H 13/52, H01H 13/14, H01H 13/06

(54) **A SWITCH BUTTON FOR AN ENDOSCOPE**

(71) Applicant: Ambu A/S, 2750 Ballerup (DK)
(72) Inventor: POMMERSHEIM, Wilhelm Franz, 86477 Adelsried (DE); GRAW, Felix Andreas, 86152 Augsburg (DE); KÖNIG, Klaus Peter, 86438 Kissing (DE)
(74) Representative: AWA Denmark A/S

(57) **Abstract**

A switch button for an endoscope handle with a housing wall (11) defining an outer handle surface and an interior compartment and at least one electrical switch (16) located on a carrier within said interior compartment. The housing wall and said carrier (17) comprises a number of through holes (17a) adapted for the switch button. The switch button comprises an operating part (19) elastically deformable under the pressure from a finger of an operator, a number of legs (18), each leg (18) comprises a spacer section (18a), a barb section (18b) and an auxiliary section (18). Furthermore, a method for mounting a switch button, a method for manufacturing a switch button, an endoscope comprising a switch button, and a system comprising a monitoring device and an endoscope are disclosed.

## Description

### Field of Invention

The invention relates to a switch button for an endoscope handle.

### Technical Background

The present invention relates to a switch button for an endoscope handle, in particular an endoscope handle with an internal compartment and a set of operating mechanisms to control features of the endoscope, such as a camera.

Endoscopes, in particular insertion endoscopes, normally comprise a proximal handle from which an insertion cord extends towards the distal end of the endoscope. The insertion cord is adapted to be inserted into a body cavity. At the distal end the insertion cord comprises an articulated bending section the bending of which can be controlled by an operator using wheels and/or levers or the like at the handle. The articulated bending section comprises a number of articulated segments including a proximal segment for attachment to a main tube forming the main body of the insertion cord, a number of intermediate segments, and a distal end segment comprising a tip part *inter alia* accommodating illumination and imaging electronics of the endoscope. In the endoscope, a working channel extends from an entry port in the handle through the insertion tube to an exit port in the distal end segment.

Using an operating member allows the operator to advance the distal tip of the endoscope to a desired location by means of a series of actions involving *inter alia* bending the bending section in a desired direction, advancing the elongated insertion cord and turning the elongated insertion cord by turning the handle which is rigidly connected thereto. Navigating a tortuous path of bends and turns to a location of interest may subject the elongated insertion cord including the distal controllable bending section to substantial forces including compression, torsion, and bending. The main body of the elongated insertion cord is essentially only bendable enough to follow the direction taken by the articulated bending section. In fact, it could be said that it is an essential part of the purpose of the elongated insertion cord to transmit the longitudinal pushing forces and rotary torsional forces from the handle to the distal end of the elongated insertion cord in order to allow these manoeuvres.

By means of an attached monitoring device, such as a monitor with a display screen, the operator is able to track movement during and after said insertion and possibly acquire pictures.

For acquiring such pictures and other purposes involving electrical control or supply signals electrical switches may be needed. In this respect it is well known to place such switches on the handle so that their operating members, i.e. switch buttons, are in positions that are easily accessible by the operators fingers during use of the endoscope.

It is known to provide endoscope handles, where the switches and switch buttons are attached to an exterior surface of the handle, e.g. as adhesive foil switches. This minimizes the risk of undesired fluids and other pollutants entering into the handle, because the handle integrity needs only be broken in one place for the passage of electrical wires, and this place may be covered by the foil of the foil switches themselves.

US2021/0169307A1 discloses an endoscope with an operating switch located inside the handle and a switch button arranged in the handle housing wall. The switch button is adapted for a multiple use endoscope, thus requiring a series of elements to securely attach the operating switch to protect electronics and other delicate parts within the handle during cleaning procedures involving aggressive cleaning fluids and high temperature and pressure from autoclaving. To achieve this, the electronics including a switch unit are sealed and fixated by a series of assembling elements, namely an elastic portion, a pressing member and a switch member. This renders the switch assembly more complex than needed for single use, i.e. disposable endoscopes, and, in consequence, has the drawback of being time consuming to assemble.

US5618212 discloses a foot-operated pedal with switches for a water vehicle. Similar to what is described for endoscopes above this pedal uses a switch foil on the exterior of a housing. The switch buttons are provided on one large cover with legs that snap into engagement with the interior wall of the housing, and seals the interior from wet or moist surroundings when used in a motorized water vehicle.

### Summary of the invention

It is a first object of the disclosure to provide a switch button which is easily manufactured and facilitates the assembly of an endoscope, in particular so as to reduce assembly time of the endoscope and reduce manufacturing costs. Furthermore, the switch button should provide good sealing properties and attachment of relevant parts of the endoscope handle while being elastically deformable under the pressure from a finger.

According to a first aspect of the disclosure this object is achieved by a switch button for an endoscope handle with a housing wall defining an outer handle surface and an interior compartment and at least one electrical switch located on a carrier within said interior compartment, wherein said housing wall and said carrier comprises a number of through holes adapted for the switch button, the switch button comprising: an operating part elastically deformable under the pressure from a finger of an operator, a number of legs, each leg comprising a spacer section, a barb section and an auxiliary section.

In this way, a switch button is adapted to fully penetrate the housing wall and the electronic switch board, thereby providing a simpler and effective assembling solution. During assembly, the auxiliary section of the legs functions as an assembly aid to allow a secure grab of the legs of the switch button in order to easily pull the legs through the through holes of the housing wall and the switch board to complete the assembly.

According to a second aspect of the disclosure the object is achieved by a method for manufacturing a switch button according to the first aspect of the disclosure wherein the switch button is manufactured by injection moulding.

According to a third aspect of the disclosure the object is achieved by a method for mounting a switch button in an endoscope comprising: providing an endoscope having a handle with a housing wall having a number of through holes and defining an outer handle surface and an interior compartment, providing a switch button according to the first aspect of the disclosure, inserting the legs of the switch button through the through holes of the handle housing from the outer handle surface, inserting the legs of the switch button into the through holes of the carrier for the electrical switch button, and passing the barb section completely through the through holes of the carrier by pulling the auxiliary section. In this way a simple assembly method is provided in which the switch button functions as a sealing mechanism. The switch button further functions as an assembly element to fixate the switch button in the interior compartment of the endoscope handle.

According to a fourth aspect of the disclosure the object is achieved by an endoscope comprising a switch button manufactured according to the second aspect of the disclosure.

According to a fifth aspect of the disclosure, the object is achieved by a system comprising a monitoring device and an endoscope according to the fourth aspect of the disclosure connectable to the monitoring device.

In an embodiment of the first aspect of the disclosure the auxiliary section has a smaller diameter than the spacer section. This facilitates the insertion of the legs into the through holes of the handle housing and the carrier.

In an embodiment the barb section completely encircles the leg. This ensures the best possible retention of the carrier.

In an embodiment the switch button further comprises a plunger adapted to operate the electric switch. This facilities assured and consistent activation of the electrical switch.

In an embodiment the plunger comprises a contact. This allows the swich to be constituted directly by conductors on the carrier, thus saving a separate electrical switch unit.

In an embodiment, the switch button is adapted to seal the through holes of the endoscope handle housing wall when mounted on the endoscope handle. This further protects the endoscope handle from undesired ingress of fluids and pollutants.

In an embodiment, the switch button is of an elastomeric material with a shore A hardness in the range 40 to 70, preferably in the range of 50 to 60. This ensures good sealing and good activation of the electric switch.

In an embodiment, the auxiliary section comprises a break zone. This may avoid a separate step of cutting away any disturbing excess length of the auxiliary section after assembly of the switch.

In an embodiment, the break zone has a cross sectional area smaller than the auxiliary section. This will weaken the auxiliary section in a desired location and ensure that after assembly the remaining length of the auxiliary section, has a suitable length.

### Brief description of the drawings

In the following, the disclosure will be described in greater detail with reference to non-limiting exemplary embodiments and the enclosed schematic drawings, on which:
FIG 1 shows a system comprising an endoscope and a monitoring device according to one embodiment of the present disclosure;
FIG. 2 shows one part of the handle housing forming the interior compartment with the electrical switch and the switch button;
FIG. 3 shows the top part of the same view of the handle in an enlarged version;
FIG 4 shows three switch buttons attached to the carrier with three electric switches;
FIG 5 and 6 show the switch button according to one embodiment of the disclosure from different angles; and
FIG 7 shows a cross-section of the switch button according to one embodiment of the disclosure.

### Detailed description

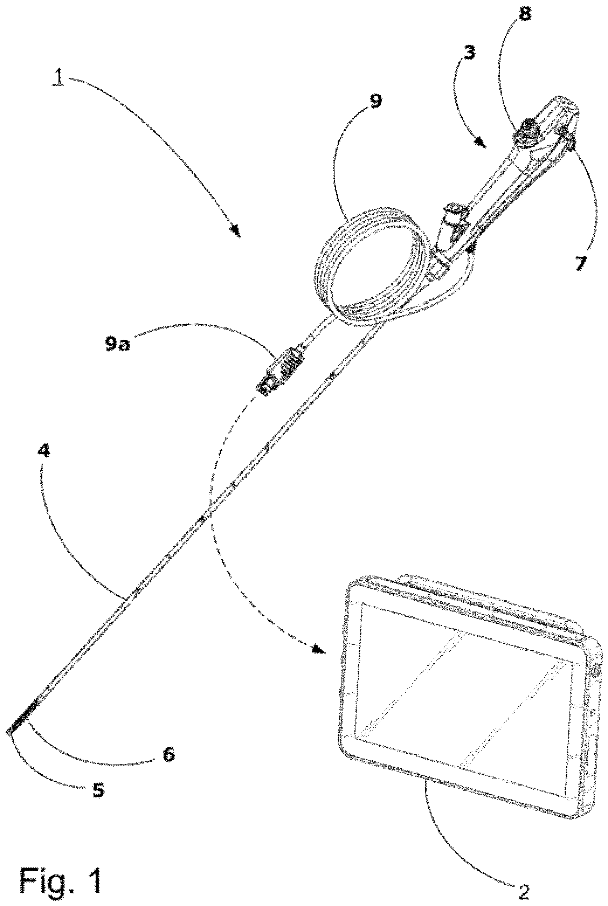

Turning first to FIG. 1, an endoscope 1 and a monitoring device 2 according to the present disclosure are shown. The endoscope 1 has a proximal end with an operating handle 3 from which an insertion cord 4 extends towards a tip part 5 at the distal end of the endoscope 1. The operating handle 3 may be ergonomically designed to be comfortably held and operated with a single hand during use by the operator. The distal end of the insertion cord 4 may constitute a bending section 6, not illustratively depicted, which is operated with a designated button 7 on the handle. The bending section may further comprise a camera which can be operated with one or more switch buttons 8 on the operating handle 3. A cable 9 having a connector 9a is provided to electrically connect the endoscope 1 with a monitoring device 2 to display images obtained with the camera.

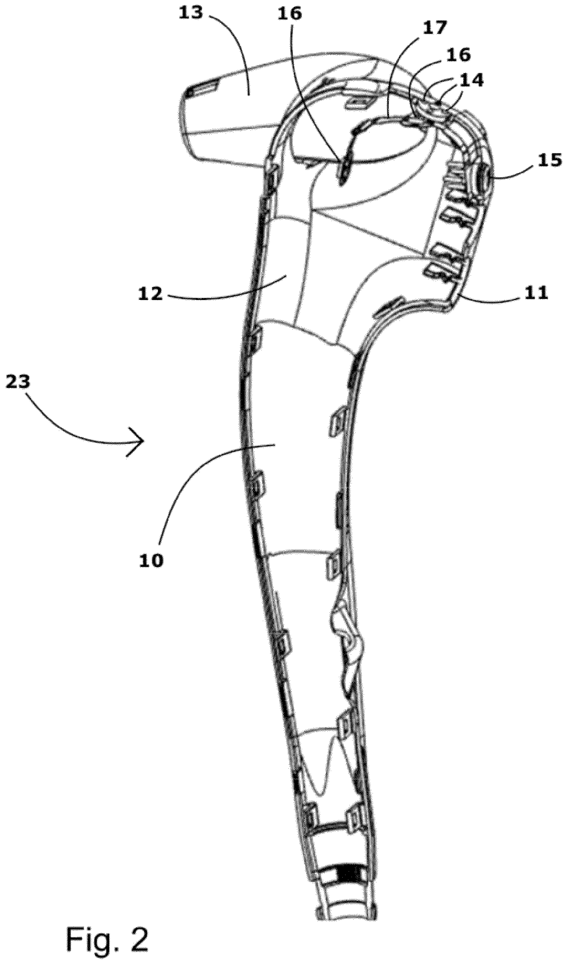

FIG. 2 shows one housing part 23 of the operating handle 3 revealing an interior compartment 10 defined by the handle housing wall 11 of the operating handle 3 together with a complementary housing part (not shown). As will be noticed the operating handle depicted in Fig. 2 differs slightly in layout from that of Fig. 1, thus illustrating that the present disclosure relates to a multitude of different layouts of operating handles 3 for endoscopes 1. The handle housing wall 11 define an inner handle surface 12 directed towards the interior compartment 10 and an outer handle surface 13 opposite the inner handle surface 12. The handle housing wall 11 comprises a number of through holes 14, some of which are configured to accommodate a switch button 15. The interior compartment 10 is configured to accommodate at least one electrical switch 16.

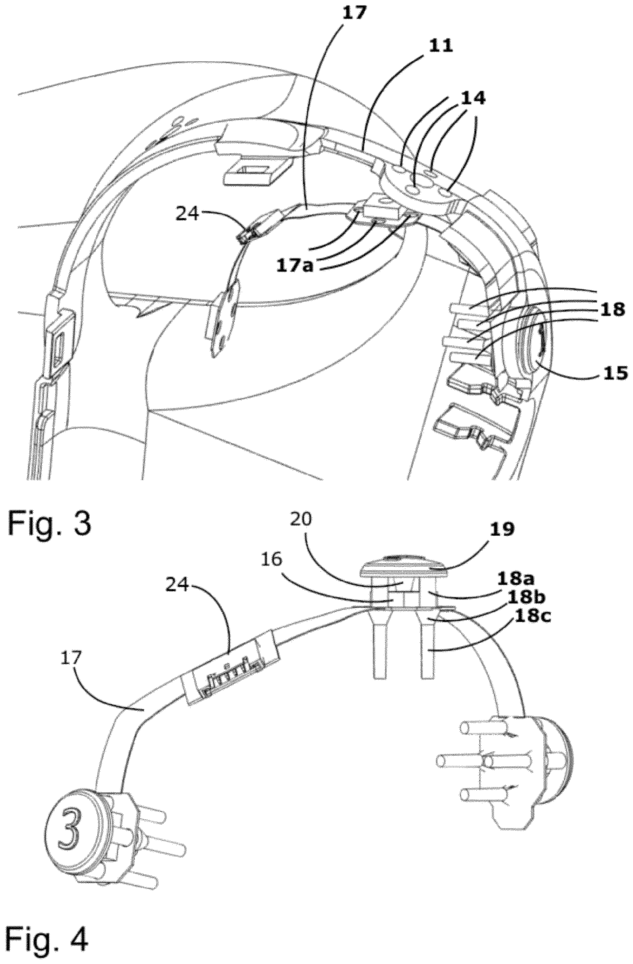

The operating handle 3 may as indicated above comprise two or more housing parts 23 fitted together during the assembly process to partly or fully enclose the interior compartment 10. As can further be seen from Fig. 2, the at least one electrical switch may be placed on a carrier 17. It should be noticed that the carrier as shown in Fig. 2 is not yet in its fully assembled state, as only one switch button 15 has been mounted. The carrier 17 is in this embodiment a flexible sheet, in particular a flexible printed circuit board, that can adjust to curvatures of the inner handle surface 12. In this respect it should be noticed, that Figs. 2 and 3 shows the carrier 17 during assembly, i.e. with only one of three switch buttons 15 in place and securing the switch assembly to the handle housing 11 wall. The carrier 17 may in another embodiment be of a more rigid material such as an epoxy based printed circuit board. In either case the carrier 17 may also incorporate a connector 24 for electrically connecting the printed circuit board to the wires or cables of the endoscope 1, e.g. leading to the monitor 2 via the cable 9.

FIG. 3 shows an enlarged version of the top part of the endoscope handle from FIG. 2 where the carrier 17 is embodied with a set of through holes 17a configured to align with the through holes 14 of the handle housing wall 11. FIG. 3 additionally shows a switch button 15 in its mounted position where the switch button 15 is assembled with the handle housing wall 11 and the carrier 17 by using the legs 18 of the switch button 15. As can best be seen in Fig. 4, the switch button 15 comprises an operating part 19. The operating part 19 is elastically deformable under the pressure from a finger of an operator. In the currently preferred embodiment of Fig. 4, the switch button 15 retains the carrier 17 together with the electrical switch 16 in position with respect to the handle housing wall 11. In this respect, the switch button 15 comprises a number of legs 18 adapted to penetrate the handle housing wall 11 and secure the switch assembly in position. Each leg comprises a spacer section 18a, a barb section 18b, and an auxiliary section 18c.

Preferably, the switch button 15 is made of an elastomeric material. The material can be a natural or synthetic rubber. In yet another embodiment it may be a silicone. The material from which the switch button 15 is made preferably has a shore A hardness in the range 40 to 70, more preferred in the range of 50 to 60. A polymer with such specifications results in a switch button that conforms to the curvatures of the outer handle surface 13 and thereby increases the sealing properties of the switch button. The elastic properties of the switch button 15 further increases the sealing properties by pressing the operating part 19 towards the outer handle surface 13 when the length of the spacer section 18a is properly selected. In a preferred embodiment the spacer section 18a has a length corresponding the combined thickness of the handle housing wall 11 and the carrier 17. In another embodiment the length of the spacer section 18a may be of a length shorter than the combined thickness of the handle housing wall 11 and the carrier 17. Consequently, the operating part 19 of the switch button 15 will exert a larger pressure on the outer handle surface 13 and provide better sealing properties. To further enhance sealing by the switch button 15 the operating part 19 will in its mounted position completely cover the through holes 14 intended for the legs 18.

In a preferred embodiment the switch button 15 has an auxiliary section 18c with crosswise dimensions smaller than the crosswise dimensions of the spacer section 18a. This will facilitate the insertion through the through holes 14 in the handle housing wall 11 and the corresponding through holes 17a arranged in the carrier 17. These holes 17a correspond to the through holes 14 in the sense that their spacing and pattern are the same, so that the through holes 17a in the carrier 17 are arranged around the electrical switch 16 and the through holes 14 in the handle housing wall are arranged around a larger central hole adapted to receive a plunger 20 of the switch button 15. Furthermore, the auxiliary part 18c may in one embodiment have a taper in the direction away from the switch button 15.

The spacer sections on the other hand have larger cross-wise dimensions, preferably adapted to fit in a sealing manner in the through holes 14 in the handle housing wall 11.

During assembly, an assembler will take the switch button 15 and easily guide the auxiliary part 18c through the through holes 14 of the handle housing wall 11, due to the narrower and preferably tapered design of the auxiliary sections 18c of the legs. This will save time during the assembling process as the assembler will easily insert and continue to guide the auxiliary part 18c through the through holes 17a of the carrier. Once the auxiliary part 18c has been successfully and effortlessly guided all the way through both sets of through holes, i.e. the through holes 14 of the handle housing wall 11 and the through holes 17a of the carrier 17, the assembler will grab the auxiliary sections 18c now located in the interior compartment 10. At this point the assembler will pull the auxiliary section 18c, thereby forcing the barb section through both sets of through holes. The spacer section 18a is now located in the through holes 14 of the handle housing wall 11 and the through holes 14 of the carrier, and ensure that carrier 17, switch button 15, and handle housing wall 11 are held tightly against each other with a suitable tension to allow the electrical switch 15 to be operated only when the operating part 19 is depressed. However, for the given ranges of hardness mentioned above the spacers will still ensure that the force is still sufficient to activate the electrical switch 15 rather than pushing the carrier 17 with the electrical switch 15 away from the handle housing wall 11 without activation the electrical switch 15. The crosswise dimension of the spacer section 18a may preferably be larger than the size of the through holes 14 of the handle housing wall 11 to further increase the sealing properties of the switch button 15. It will still be possible to force the operating part 19 to be in close contact with the outer handle surface 13 due to the elastic properties of the spacer section 18a.

After assembly the auxiliary sections 18c are no longer needed and may be removed e.g. by cutting away or otherwise removing any excess length thereof that could conflict with other internal parts of the endoscope 1, e.g. pull wires and operating members thereof within the operating handle 3.

In the embodiment shown in FIG. 4-7 the switch button 15 has a plunger 20. The plunger 20 extends from the operating part 19 of the switch button 15. The plunger 20 may extend in a direction that is parallel with the legs 18 of the switch button 15. In this embodiment the operating part 19 of the switch button is of a dome shape. This shape helps keeping the through holes 14 completely sealed during operation of the switch button and, combined with the elastic properties of the material, provide a comfortable return of the operating part 19 to its original shape when pressure is released after operating the switch button 15. In another embodiment the operating part 19 may be square in its profile. This is, however, less preferred as round holes 14, 17a and legs are easier to manufacture.

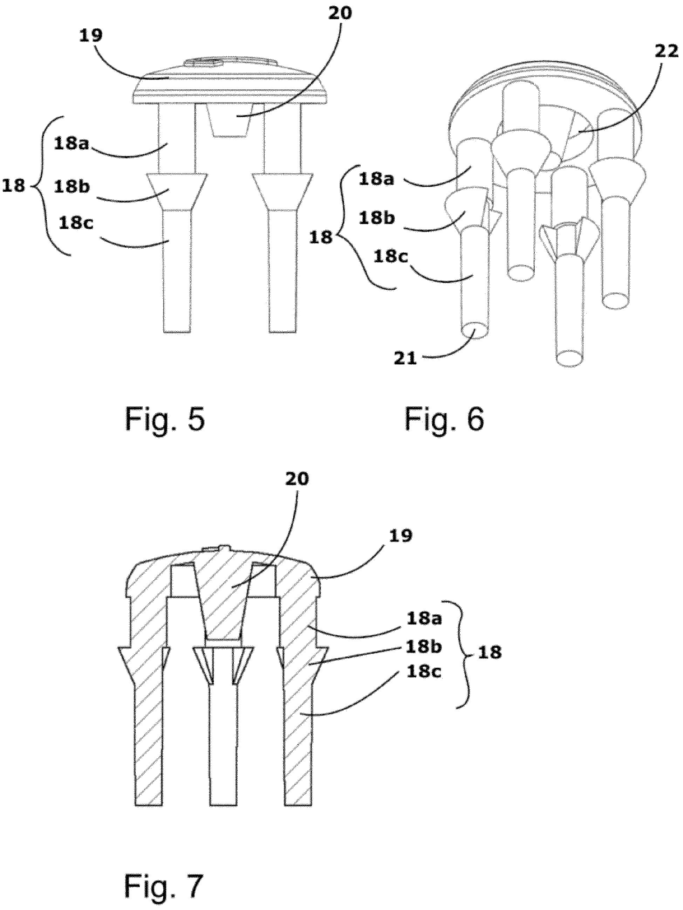

Fig. 6 shows an angled view of the switch button 15. In this view the end part 21 of the leg 18 can be seen to have a circular transversal cross-section. In this preferred embodiment all parts of the leg i.e. the spacer section 18a, the barb section 18b, and the auxiliary section 18c all have circular cross-sectional areas. In another embodiment the transversal cross-section of the legs 18 may be square or of another more complex shape.

The barb section 18b of the leg 18 may in one embodiment completely encircle the leg. In a preferred embodiment the barb section 18b partly encircles the leg 18 to an extend in the range of 180° - 360°. This encircling may be continuous or in a number of discrete barbs e.g. as circular sectors. The barbs are preferably conical to easily guide them through the through holes 14 of the outer handle surface 11 and the through holes of the carrier 17a during assembly. Consequently, the barbs 18b will not move reversely through the through holes 14 and 17a once mounted and the switch button 15 along with the carrier 17 will be securely attached to the handle housing wall 11.

In one embodiment the operating part may have a circular groove 22 surrounding the plunger 20 as shown in the embodiment of Fig. 6 and Fig. 7. This groove 22 makes it easier to push the plunger 20 down when operating the switch button and contributes to a more comfortable and less energy demanding operation.

The switch button 15 is moulded as one unitary item, and is preferably manufactured with injection moulding. This requires a moulding chamber that captures all the details of the switch button 15, with as few moulding parts as possible. The switch button is preferably moulded with two moulding parts a concave and a convex part. Due to the elasticity of the material with a Shore A hardness in the range of 40 to 70, it will in fact be possible to retract the convex mould part from the concave mould port, even if the barb sections 18b are undercut. However, in order for the convex part to reach and form the plunger 20 and the groove 22 around it is preferred to form the barbs second 18b with cut-out. The barb 18b will for this reason only partly encircle the leg to allow room for the moulding part.

In one embodiment the auxiliary section 18c of the leg may comprise a break zone. The break zone may have a cross-sectional area smaller than the cross-sectional area of the auxiliary section 18c not comprising the break zone. In one embodiment the break zone may be a structural weakening in the form of a slit.

It should be emphasized that the embodiments shown are used for example purposes only and should not be used to limit the scope of the invention.

In particular it can be envisaged that the plunger 20 carries a conducting contact part adapted to engage conductors directly on a printed circuit board constituting the carrier 17, thus obviating the need for a switch.

Furthermore, the skilled person will appreciate that although described in the context of an endoscope the disclosed switch button may be used in a multitude of other devices where a simple way of securing a switch assembly located behind a wall is desired.

## Claims

1. A switch button for an endoscope handle with a housing wall defining an outer handle surface and an interior compartment and at least one electrical switch located on a carrier within said interior compartment, wherein said housing wall and said carrier comprises a number of through holes adapted for the switch button, the switch button comprising:
an operating part elastically deformable under the pressure from a finger of an operator,
a number of legs, each leg comprising a spacer section, a barb section and an auxiliary section.

2. A switch button according to claim 1, wherein the auxiliary section has a smaller diameter than the spacer section.

3. A switch button according to any one of the preceding claims, wherein the barb section completely encircles the leg.

4. A switch button according to any one of the preceding claims, wherein the switch button further comprises a plunger adapted to operate the electric switch.

5. A switch button according to claim 4, wherein the plunger comprises a contact.

6. A switch button according to any one of the preceding claims, wherein the switch button is adapted to seal the through holes of the endoscope handle housing wall when mounted on the endoscope handle.

7. A switch button according to any one of the preceding claims, wherein the switch button is of an elastomeric material with a shore A hardness in the range 40 to 70, preferably in the range of 50 to 60.

8. A switch button according to any of one of the preceding claims, wherein the auxiliary section comprises a break zone.

9. A switch button according to claim 8, wherein the break zone has a cross sectional area smaller than the auxiliary section.

10. A method for manufacturing a switch button according to any one of the preceding claims, wherein the switch button is manufactured by injection moulding.

11. A switch button according to claim 10, wherein the switch button is integrally moulded as one unitary item.

12. A method for mounting a switch button according to any of the preceding claims comprising:
providing an endoscope having a handle with a housing wall having a number of through holes and defining an outer handle surface and an interior compartment,
providing a switch button according to any one of claims 1 to 9,
inserting the legs of the switch button through the through holes of the handle housing from the outer handle surface,
inserting the legs of the switch button into the through holes of the carrier for the electrical switch button, and
passing the barb section completely through the through holes of the carrier by pulling the auxiliary section.

13. An endoscope comprising a switch button manufactured according to the method of claim 10.

14. A system comprising a monitoring device and an endoscope according to claim 13 connectable to said monitoring device.
